Europäisches Patentamt

European Patent Office

Office européen des brevets

(11) Veröffentlichungsnummer : **0 047 523**
**B1**

(12) **EUROPÄISCHE PATENTSCHRIFT**

(45) Veröffentlichungstag der Patentschrift :
30.01.85

(51) Int. Cl.⁴ : **C 12 N 9/50**, B 01 D 15/08

(21) Anmeldenummer : 81107031.7

(22) Anmeldetag : 07.09.81

(54) **Verfahren zur selektiven Abtrennung von Endoproteasen.**

(30) Priorität : 10.09.80 DE 3034043

(43) Veröffentlichungstag der Anmeldung :
17.03.82 Patentblatt 82/11

(45) Bekanntmachung des Hinweises auf die Patenterteilung : 30.01.85 Patentblatt 85/05

(84) Benannte Vertragsstaaten :
AT BE CH DE FR GB IT LI LU NL SE

(56) Entgegenhaltungen :
FR-A- 2 210 620
GB-A- 2 083 477
THROMBOSIS AND HAEMOSTASIS, Band 38, Nr. 1,
1977, Seite 144 R.F. HIGHSMITH et al.: "Human
plasmin and protease inhibitors: interaction in a
whole plasma system"
BIOLOGICAL ABSTRACTS, Band 66, 1978, Nr. 15636;
S.S. TWINING et al.: "Large scale separation of
protease inhibitors from malignant human breast
tissue"

(73) Patentinhaber : BOEHRINGER MANNHEIM GMBH
Patentabteilung, Abt. E Sandhofer Strasse 112-132
Postfach 31 01 20
D-6800 Mannheim 31 Waldhof (DE)

(72) Erfinder : Schrenk, Jürgen, Dr.
Kiefernstrasse 4
D-8120 Weilheim (DE)
Erfinder : Wunderwald, Peter, Dr.
Schulstrasse 6
D-8121 Haunshofen (DE)

(74) Vertreter : Weickmann, Heinrich, Dipl.-Ing. et al
Patentanwälte Dipl.-Ing. H.Weickmann Dipl.-Phys.Dr.
K.Fincke Dipl.-Ing. F.A.Weickmann Dipl.-Chem. B.
Huber Dr.-Ing. H. Liska Dipl.-Phys.Dr. J. Prechtel
Postfach 860820
D-8000 München 86 (DE)

## Beschreibung

Die Erfindung betrifft die selektive Abtrennung von Endoproteasen aus wässrigen Lösungen, insbesondere ihre Abtrennung von anderen biologisch aktiven Proteinen in wässriger Lösung.

Endoproteasen sind proteolytische Enzyme, welche Protein an bestimmten Stellen in der Mitte der Kette spalten und das Kettenende nicht angreifen. Beispiele für Endopeptidasen sind Pepsin, Trypsin, Chymotrypsin, Collagenase, Papain, Ficin, Clostripain, Subtilisin, Elastase und Bromelain, neben zahlreichen anderen.

Ein besonderes Problem ist das Vorhandensein derartiger Endoproteinasen bei Verfahren zur Gewinnung biologisch aktiver Protein, insbesondere wenn ein biologisches Ausgangsmaterial verwendet wird, welches relativ reich an solchen Endoproteinasen ist. Da sich diese Endoproteinasen häufig sehr schwer von den anderen Proteinen abtrennen lassen, und die Reinigung und Fraktionierung von biologisch aktiven Proteinen meistens unter Bedingungen erfolgt, bei denen die Endoproteasen aktiv sind, stören letztere die Reinigung der anderen biologisch aktiven Proteine sehr und sind großenteils für die Verluste an biologischer Aktivität verantwortlich, die bei derartigen Reinigungsoperationen meistens auftreten. Ein Verfahren, welches es ermöglicht, die Endoproteinasen selektiv abzutrennen, würde daher die Praktikabilität vieler Methoden zur Reinigung biologisch aktiver Protein wesentlich verbessern.

Obwohl jedoch ein Bedarf an einem solchen Verfahren zur spezifischen Abtrennung von Endoproteinasen aux wässrigen Lösungen besteht, war es bisher nicht gelungen, ein allgemein befriedigendes derartiges Verfahren aufzufinden.

Dieses Problem wird durch die Erfindung gelöst.

Das erfindungsgemäße Verfahren zur selektiven Abtrennung von Endoproteinasen aus wässrigen Lösungen ist dadurch gekennzeichnet, daß man die Lösung mit einem in fester Phase vorliegenden Komplex von alpha$_2$-Makroglobulin und einem zweiwertigen Metall aus der Gruppe Zn, Co, Ni und Cu behandelt und dann die feste Phase abtrennt. Die Endoproteinasen werden selektiv an den alpha$_2$-Makroglobulin-Metall-Chelat-Komplex fixiert und können aus der wässrigen Lösung mit der festen Phase abgetrennt werden. Alpha$_2$-Makroglobulin und seine Eigenschaften sind bekannt, beispielsweise aus TIBS 5, 43-47 (1980). Alpha$_2$-Makroglobulin, im Folgenden als $\alpha_2$M bezeichnet, ist ein universeller Proteaseninhibitor, von dem bekannt ist, daß er mit den Proteasen einen Komplex bildet, bei dem er eine drastische Veränderung seiner Struktur erleidet (siehe z. B. Figur 1 von TIBS loc. cit.).

Weiter war bekannt, aus Anal. Biochem. 99, 415-420 (1979), daß $\alpha_2$M über eine Säule mit trägergebundenem Zn-Chelat gereinigt werden kann. Dabei wurde beobachtet, daß keine $\alpha_2$M-Protease-Komplexe auftraten, die für die Proteasenbindung erforderliche Strukturänderung des $\alpha_2$M-Moleküls also bei Bindung an das Zn-Chelat nicht möglich war. Überraschenderweise wurde jedoch nunmehr gefunden, daß trotzdem Endoproteasen selektiv an $\alpha_2$M-Metallchelat-Komplexen bestimmter Metalle binden und auf diese Weise von Begleitsubstanzen, insbesondere anderen Proteinen, abgetrennt werden können. Diese Feststellung widerspricht somit den bisherigen Kenntnissen über die Eigenschaften des $\alpha_2$M.

Das erfindungsgemäße Verfahren eignet sich zur selektiven Abtrennung aller Endoproteinasen, die durch $\alpha_2$M auch gehemmt werden. Es ermöglicht damit auch die Trennung von Endoproteinasen, die durch $\alpha_2$M gehemmt werden, und solchen Endoproteinasen, die durch $\alpha_2$M nicht gehemmt werden.

Als Trägermaterial für die feste Phase eignen sich vor allem Kationenaustauscher, welche die oben gennante Gruppe von zweiwertigen Metallen zu binden vermögen. Bevorzugt werden Kationenaustauscher, welche die Carboxymethylamino-Gruppe tragen, beispielsweise Carboxymethylamino-Agarose oder Iminoessigsäure-Sepharose.

Die Herstellung des beim erfindungsgemäßen Verfahrens verwendeten, in fester Phase vorliegenden Komplexes von $\alpha_2$M und zweiwertigem Metall, erfolgt gemäß einer ersten Ausführungsform der Erfindung so, daß man das $\alpha_2$M in der die abzutrennenden Endoproteinasen enthaltenden wässrigen Lösung auflöst und diese Lösung mit einem Kationenaustauscher behandelt, der mit dem zweiwertigen Metall, also Zn, Co, Ni oder/und Cu, beladen ist. Alternativ ist es auch möglich, zuerst $\alpha_2$M in Lösung mit dem mit zweiwertigen Metall beladenen Kationenaustauscher zu kontaktieren, wobei das $\alpha_2$M an die feste Phase gebunden wird, und anschließend die feste Phase mit der proteasehaltigen Lösung zu mischen.

Unabhängig davon welche der beiden obigen Ausführungsformen der Erfindung angewendet wird, kann man die proteasehaltige Lösung über die feste Phase chromatographieren oder die proteasenhaltige Lösung mit der festen Phase einfach mischen und anschließend wiederum daraus entfernen (Batch-Verfahren). Wie aus der oben beschriebenen ersten Ausführungsform der Erfindung ersichtlich ist, wird auch der bereits gebildete Komplex $\alpha_2$M-Endoprotease an das trägergebundene zweiwertige Metall fixiert. Die Bindung ist quantitativ, und die erfindungsgemäß behandelte Lösung ist anschließend frei von Endopeptidasen, die durch $\alpha_2$M hemmbar sind.

Zur Regenerierung der festen Phase wird diese mit Salzlösungen geeigneter Konzentration gewaschen. Geeignet sind beispielsweise 0,05 bis 0,5 M, vorzugsweise 0,08 bis 0,2 M Natriumacetat von pH 4 bis 5, vorzugsweise 4,3 bis 4,7, mit einem Gehalt von 0,5 bis 1 M, vorzugsweise 0,7 bis 0,9 M NaChl. Ein anderes Beispiel ist 0,5 bis 2 N NaOH. Weiter ist auch Dimethylarsin-Puffer 0,01 bis 2,0 M, vorzugsweise 0,02 bis 0,05 M, pH 4 bis 6, vorzugsweise 4,7 bis 5,3, mit einem NaCl-Gehalt von 0,1 bis 1 M, vorzugsweise 0,5 bis 0,8 M geeignet. Der auf diese Weise eluierte Komplex $\alpha_2$M-Endoproteinase läßt sich nur unter Denaturierung, z. B. Reduktion, zerlegen.

Zur Herstellung des festen metallkomplexhaltigen Trägermaterials wird eine Lösung des jeweiligen zweiwertigen Metalls, welches eingesetzt werden soll, in Form eines geeigneten Salzes, vorzugsweise als Salz einer Mineralsäure, wie als Sulfat oder Chlorid, mit einen Kationenaustauscher, zweckmäßig einem Kationenaustauscherharz, zusammengebracht. Die Konzentration der Metallsalzlösung beträgt vorzugsweise 3 bis 8 mg/ml, der pH-Wert sollte zwischen 4 und 6,5 liegen. Es können jedoch auch Lösungen außerhalb der angegebenen Bereiche verwendet werden, jedoch können hierbei Verluste auftreten, und die Reinigung kann erschwert sein. Nach der Bindung der Metallionen an den Träger wird zweckmäßig mit Puffer, beispielsweise 2-10 Vol 0,05 bis 0,5 M Acetat-Puffer, welcher 0,1 bis 0,5 M NaCl enthält und einen pH-Wert zwischen 4 und 6 aufweist, gründlich gewaschen.

Wird das zu fixierende $\alpha_2$M als solches an den Träger gebunden, so setzt man es zweckmäßig in Form einer Lösung in einem geeigneten Puffer ein. Geeignet sind im allgemeinen Pufferkonzentrationen zwischen 0,01 und 0,05 M, bevorzugt werden 0,02 bis 0,03 M. Der pH-Wert sollte zwischen 5,5 und 9 liegen, vorzugsweise zwischen 6,0 und 8. Alle in diesem Bereich puffernden Puffersubstanzen können verwendet werden, jedoch werden Natrium- und Kalium-phosphat-Puffer bevorzugt. Im allgemeinen können pro ml Trägervolumen 10 bis 100 U $\alpha_2$M fixiert werden.

Die endoproteinasehaltige Lösung sollte einen pH-Wert zwischen 6 und 8,5 aufweisen, der Salzgehalt sollte 0,1 M nicht übersteigen. Als Puffersubstanzen kommen wiederum alle in diesem Bereich puffernden in Betracht, bevorzugt werden Phosphat-Puffer und Tris-Puffer.

Wird das Verfahren der Erfindung durchgeführt, indem zuerst die zu behandelnde Endoproteinasenlösung mit $\alpha_2$M versetzt und die Lösung erst danach über den metallchelathaltigen Träger gegeben wird, so gelten bzgl. der Salzkonzentration, des pH-Wertes und der Puffersubstanzen die obigen Ausführungen entsprechend.

Das erfindungsgemäße Verfahren macht es möglich, störende Endoproteinasen rasch und einfach aus diese enthaltenden Lösungen zu entfernen und auf diese Weise vor allem biologisch aktive Proteine gegen Aktivitätsverluste infolge der Einwirkung von Endoproteinasen zu schützen, beziehungsweise zu stabilisieren. Ferner eignet sich das Verfahren der Erfindung auch zur Abtrennung der durch $\alpha_2$M hemmbaren Endoproteasen von anderen Endoproteasen und Exoproteasen.

Erfindungsgegenstand ist weiter ein Mittel zur Durchführung des erfindungsgemäßen Verfahrens, welches aus einem festen Trägermaterial besteht, welches mit einem Komplex aus $\alpha_2$M und einem zweiwertigen Metall aus der Gruppe Zn, Co, Ni und Cu beladen ist. Vorzugsweise besteht der Träger aus einem Kationenaustauscherharz, mehr bevorzugt aus Carboxymethylamino-Gruppen enthaltender Agarose. Jedoch können auch andere Trägermaterialien, wie sie für die Fixierung von biologisch aktiven Proteinen gebräuchlich sind, in gleicher Weise verwendet werden. Wesentlich ist nur, daß sie zweiwertigen Metallionen aufziehen.

Die folgenden Beispiele erläutern die Erfindung weiter.

Beispiele

Beispiel 1

Bis-Carboxymethylamino-Agarose wird mit dem 5fachen Volumen $ZnCl_2$-Lösung (3 mg/ml, pH 6,3) beladen und anschliessend mit dem 3fachen Volumen Acetatpuffer gewaschen (0,05 M Na-Acetat + 0,15 M NaCl · pH 5,0). An diesen Zn-Chelat-Träger wird dann in Phosphatpuffer (0,02 M Na-Phosphate + 0,15 M NaCl · pH 6,0) alpha$_2$-M absorbiert. Die fertige alpha$_2$-M-Säule wird mit dem 10-15-fachen Volumen Phosphatpuffer gewaschen.

Über 1 ml dieses Adsorbens werden 2 ml dialysierter He-feextrakt in 0,05 M K-Phosphat, pH 7,0 + $10^{-3}$ M EDTA + $10^{-3}$ m MCE chromatographiert. Der Durchlauf wird mit 1 mM EDTA und 0,2 mM Azid und 50 % Glycerin versetzt. Die alpha-Glucosidase-Aktivität nach 11 Tagen Belastung bei 33 °C beträgt noch immer 100 % (unbehandelte Kontrolle : 73 %), nach 19 Tagen Belastung noch 92 % (unbehandelte Kontrolle : 32 %). Der alpha-Glucosidase-Test wird durchgeführt wie beschrieben in H. U. Bergmeyer, Methoden der enzymatischen Analyse, Verlag Chemie, Weinheim/Bergstraße, S. 488 (1974).

Beispiel 2

Eine aus 206 Ltr. Kulturfiltrat von Lysobacter enzymogenes ssp. enzymogenes, DSM 1895 (ATCC 27996) durch Ammonsulfatfraktionierung und Sephadex-G-100-Chromatographie vorgereinigte Präparation (dieses Verfahren ist in der gleichzeitig unter der int. Nr. 2399 eingereichten Anmeldung näher beschrieben), welche verschiedene durch $\alpha_2$M hemmbare Endoproteinasen enthält, wird mit dem in Beispiel 1 beschriebenen $\alpha_2$M-Zn-Komplex behandelt. Hierzu werden 110 ml dieses Trägermaterials in 3 cm ∅ Säule, Länge 17,5 cm eingefüllt und mit 0,05 M Trispuffer, pH 8,0 gewaschen, bis sich kein Protein im Durchlauf befindet. Dann wird das vorgereinigte Kulturfiltratpräparat aufgezogen und mit 0,05 M Tris, pH 8,0, nachgewaschen. Nur die bisher nicht beschriebene Lys-C-Proteinase läuft durch, alle anderen Proteinasen werden zurückgehalten.

# 0 047 523

## Ansprüche

1. Verfahren zur selektiven Abtrennung von Endoproteasen aus wässrigen Lösungen, dadurch gekennzeichnet, daß man die Lösungen mit einem in fester Phase vorliegenden Komplex von alpha$_2$-Makroglobulin mit einem zweiwertigen Metall aus der Gruppe Zn, Co, Ni und Cu behandelt und dann die feste Phase abtrennt.

2. Verfahren nach Anspruch 1, dadurch gekennzeichnet, daß man das alpha$_2$-Makroglobulin in der wässrigen Lösung auflöst und die erhaltene Lösung mit einem Kationenaustauscher behandelt, der mit dem zweiwertigen Metall beladen ist.

3. Verfahren nach Anspruch 1, dadurch gekennzeichnet, daß man das alpha$_2$-Makroglobulin mit einem mit dem zweiwertigen Metall beladenen Kationenaustauscher kontaktiert und dann mit der proteasehaltigen Lösung mischt.

4. Verfahren nach einem der Ansprüche 1 bis 3, dadurch gekennzeichnet, daß man die proteasehaltige Lösung über die feste Phase chromatographiert.

5. Verfahren nach einem der Ansprüche 1 bis 3, dadurch gekennzeichnet, daß man die feste Phase in die proteasehaltige Lösung gibt und danach wieder daraus entfernt.

6. Mittel zur Durchführung des Verfahrens nach Anspruch 1, dadurch gekennzeichnet, daß es aus einem festen Trägermaterial besteht, welches mit einem Komplex aus alpha$_2$-Makroglobulin und einem zweiwertigen Metall aus der Gruppe Zn, Co, Ni und Cu beladen ist.

7. Mittel nach Anspruch 6, dadurch gekennzeichnet, daß das Trägermaterial aus Carboxymethylamino-Agarose besteht.

## Claims

1. Process for the selective separation of endoproteases from aqueous solutions, characterised in that one treats the solutions with a complex, present in the solid phase, of alpha$_2$-macroglobulin with a divalent metal of the group Zn, Co, Ni and Cu and then separates off the solid phase.

2. Process according to claim 1, characterised in that one dissolves the alpha$_2$-macroglobulin in the aqueous solution and treats the solution obtained with a cation exchanger which is loaded with the divalent metal.

3. Process according to claim 1, characterised in that one contacts the alpha$_2$-macroglobulin with a cation exchanger loaded with the divalent metal and then mixes with the protease-containing solution.

4. Process according to one of claims 1 to 3, characterised in that one chromatographs the protease-containing solution over the solid phase.

5. Process according to one of claims 1 to 3, characterised in that one adds the solid phase to the protease-containing solution and thereafter again removes it therefrom.

6. Agent for carrying out the process according to claim 1, characterised in that it consists of a solid carrier material which is loaded with a complex of alpha$_2$-macroglobulin and of a divalent metal of the group Zn, Co, Ni and Cu.

7. Agent according to claim 6, characterised in that the carrier material consists of carboxymethylaminoagarose.

## Revendications

1. Procédé pour la séparation sélective d'endoprotéases de solutions aqueuses, caractérisé en ce qu'on traite les solutions avec un complexe se trouvant en phase solide d'alpha$_2$-macroglobuline avec un métal divalent du groupe Zn, Co, Ni et Cu, puis en ce qu'on sépare la phase solide.

2. Procédé suivant la revendication 1, caractérisé en ce qu'on dissout l'alpha$_2$-macroglobuline dans la solution aqueuse et on traite la solution obtenue avec un échangeur de cations qui est chargé avec le métal divalent.

3. Procédé suivant la revendication 1, caractérisé en ce qu'on met en contact l'alpha$_2$-macroglobuline avec un échangeur de cations chargé avec le métal divalent, puis on le mélange avec la solution contenant la protéase.

4. Procédé suivant l'une des revendications 1 à 3, caractérisé en ce qu'on chromatographie la solution contenant la protéase sur la phase solide.

5. Procédé suivant l'une des revendications 1 à 3, caractérisé en ce qu'on introduit la phase solide dans la solution contenant la protéase, puis en ce qu'on l'en élimine ensuite à nouveau.

6. Agent pour l'exécution du procédé suivant la revendication 1, caractérisé en ce qu'il se compose d'une matière de support solide qui est chargée avec un complexe d'alpha$_2$-macroglobuline et un métal divalent du groupe Zn, Co, Ni et Cu.

7. Agent suivant la revendication 6, caractérisé en ce que la matière de support se compose de carboxyméthylamino-agarose.